(19) Europäisches Patentamt
European Patent Office
Office européen des brevets

(11) Veröffentlichungsnummer: **0 000 335**
**B1**

(12) # EUROPÄISCHE PATENTSCHRIFT

(45) Veröffentlichungstag der Patentschrift: **07.10.81**

(51) Int. Cl.³: **C 07 D 239/48,**
A 61 K 31/505

(21) Anmeldenummer: **78100183.9**

(22) Anmeldetag: **19.06.78**

(54) **N-Pyrimidinyl-imidsäureester, Verfahren zu ihrer Herstellung und diese enthaltende Arzneimittel.**

(30) Priorität: **06.07.77 DE 2730468**

(43) Veröffentlichungstag der Anmeldung:
**24.01.79 Patentblatt 79/2**

(45) Bekanntmachung des Hinweises auf die Patenterteilung:
**07.10.81 Patentblatt 81/40**

(84) Benannte Vertragsstaaten:
**BE CH DE FR GB LU NL SE**

(56) Entgegenhaltungen:
**Keine**

(73) Patentinhaber: **BASF Aktiengesellschaft**
**Carl-Bosch-Strasse 38**
**D-6700 Ludwigshafen (DE)**

(72) Erfinder: **Scharwaechter, Peter, Dr.**
**An der Duene 9**
**D-2082 Moorrege (DE)**
Erfinder: **Gutsche, Klaus, Dr.**
**Ehmschenkamp 5**
**D-2084 Rellingen (DE)**
Erfinder: **Kohlmann, Wilhelm, Dr.**
**An der Duene 6**
**D-2082 Moorrege (DE)**
Erfinder: **Kroemer, Gerd, Dr.**
**verstorben, zuletzt wohnhaft Kaltenweise 100a**
**D-2200 Elmshorn (DE)**

**EP 0 000 335 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

N-Pyrimidinyl-imidsäureester, Verfahren zu ihrer Herstellung und diese enthaltende Arzneimittel

Die Erfindung betrifft Imidsäureester der allgemeinen Formel I

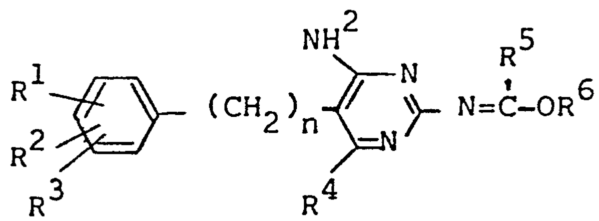

(I)

in der $R^1$, $R^2$ und $R^3$, die gleich oder verschieden voneinander sein können, Wasserstoff, Methyl, Methoxy oder Chlor bedeuten, $R^4$ Wasserstoff oder Alkyl mit 1—4 C-Atomen bedeutet, $R^5$ Alkyl mit 1—6 C-Atomen oder Benzyl bedeutet, $R^6$ Alkyl mit 1—4 C-Atomen oder Benzyl bedeutet, und n gleich 0 oder 1 sein kann.

Bevorzugt stehen die Substituenten $R^1$, $R^2$ und $R^3$ in der 3-, 4- und 5-Stellung des Benzolringes.

Unter den Verbindungen der allgemeinen Formel I sind diejenigen bevorzugt, in denen $R^4$ Wasserstoff oder Äthyl bedeutet.

Die Herstellung der erfindungsgemäßen Verbindungen der allgemeinen Formel I geschieht nach den für die Herstellung von Imidsäureestern üblichen Methoden, wie sie u.a. im Houben-Weyl, "Methoden der organischen Chemie", Band 6/3 beschrieben sind, indem man eine Verbindung der allgemeinen Formel II

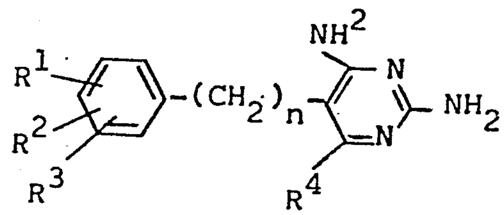

(II)

worin $R^1$, $R^2$, $R^3$, $R^4$ und n die oben angegebene Bedeutung haben, mit einem Orthocarbonsäureester der allgemeinen Formel III

$$R^5—C(OR^6)_3 \qquad (III)$$

in der $R^5$ und $R^6$ die oben genannte Bedeutung haben, umsetzt.

Die Herstellung der Verbindungen der allgemeinen Formel I kann mit oder ohne Lösungsmittel erfolgen, wobei im letzteren Falle mit einem Überschuß des eingesetzten Orthocarbonsäureesters gearbeitet wird. Als mögliche Lösungsmittel eignen sich beispielsweise Dimethylformamid oder Dioxan. Die Reaktionstemperaturen liegen zwischen 0 und 150°C, bevorzugt zwischen 50 und 100°C bzw. bei Temperaturen bis zum Siedepunkt des verwendeten Lösungsmittels oder Orthocarbonsäureesters. Die Umsetzung kann gegebenenfalls in Gegenwart katalytischer Mengen einer Säure, wie beispielsweise Salzsäure, durchgeführt werden.

Verwendet man beispielsweise 2,4-Diamino-5-(3,4,5-trimethoxybenzyl)-pyrimidin und Orthoessigsäuretriäthylester als Ausgangsstoffe, so kann der Reaktionsverlauf durch folgendes Formelschema wiedergegeben werden:

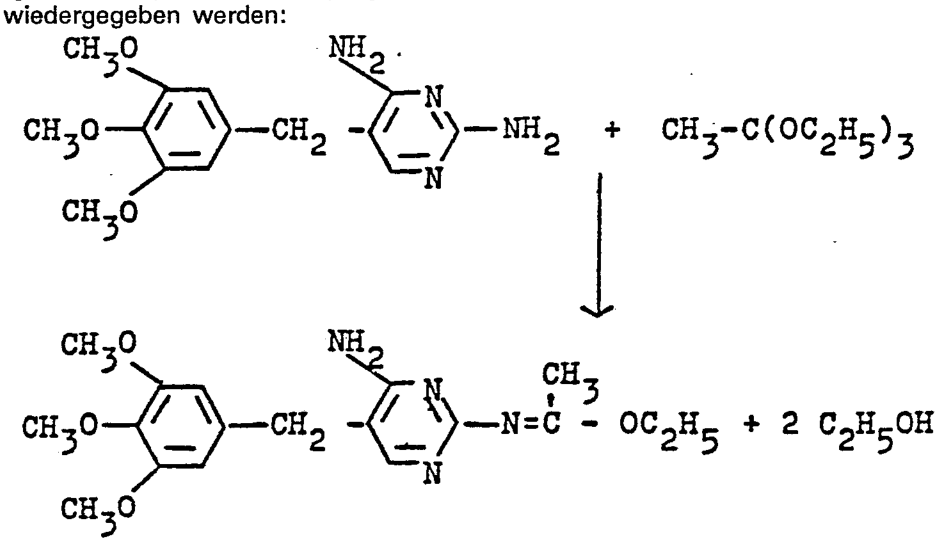

Die erfindungsgemäßen Verbindungen der allgemeinen Formel I sind antimikrobiell wirksam bei durch Bakterien und Protozoen hervorgerufenen Krankheiten und potenzieren, kombiniert mit Sulfonamiden, deren antimikrobielle Wirkung. Sie können daher beispielsweise bei bakteriellen Erkrankungen der Atmungsorgane, Verdauungsorgane und Harnwege sowie bei Hals-, Nasen-, Ohreninfektionen und allgemein systemischen Infektionskrankheiten und bei Malaria verwendet werden.

Solche Sulfonamide sind beispielsweise:
2-Sulfanilamido-pyridin, 2-Sulfanilamido-thiazol, 2-Sulfanilamido-pyrimidin, 2-Sulfanilamido-4-methyl-pyrimidin, 2-Sulfanilamido-4,6-dimethyl-pyrimidin, 4-Sulfanilamido-2,6-dimethyl-pyrimidin, 5-Sulfanilamido-3,4-dimethyl-isoxazol, 3-Sulfanilamido-6-methoxy-pyridazin, 3-Sulfanilamido-6-chlor-pyridazin, 4-Sulfanilamido-2,6-dimethoxy-pyrimidin, 3-Sulfanilamido-2-phenyl-pyrazol, 2-Sulfanilamido-5-methyl-pyrimidin, 2-Sulfanilamido-5-methoxy-pyrimidin, 2-Sulfanilamido-5-methyl-isoxazol, 2-Sulfanilamido-4,5-dimethyl-oxazol, 2-Sulfanilamido-3-methoxy-pyrazin, 4-Sulfanilamido-5,6-dimethoxy-pyrimidin, 4-Sulfanilamido-3-methoxy-1,2,5-thiadiazol, 4-Aminobenzol-sulfonyl-guanidin.

Die Verbindungen der allgemeinen Formel I können mit den beispielhaft genannten Sulfonamiden in verschiedenen Mischungsverhältnissen kombiniert werden, wobei das Verhältnis Verbindung der allgemeinen Formel I zu Sulfonamid von 1:10 bis 5:1 variieren kann. Bevorzugte Mischungsverhältnisse sind 1:1 bis 1:5. Dabei kommen in der Regel als Dosierung 20 bis 500 mg eines Wirkstoffs der allgemeinen Formel I in Betracht.

Zum Wirksamkeitsnachweis wurden erfindungsgemäße Substanzen im Tierversuch am Modell der sogenannten Aronson-Sepsis, wobei mit Streptococcus agalactiae infiziert wird, geprüft und mit dem bekannten Trimethoprim verglichen. Hierzu wurden Gruppen von je 30 wieblichen Mäusen mit einer tödlichen Dosis von Streptococcus agalactiae 7941 infiziert und 2 Stunden nach der Infektion mit einer Mischung von 300 mg 2-Sulfanilamido-4,5-dimethyloxazol + 60 mg einer der erfindungsgemäßen Substanzen behandelt. Außer einer nicht behandelten Kontrollgruppe wurde eine zweite Gruppe mit dem als Referenzsubstanz dienenden Gemisch von 300 mg 2-Sulfanilamido-4,5-dimethyloxazol + 60 mg Trimethoprim behandelt. Nach 44 Stunden wurde die Zahl der überlebenden Tiere bestimmt und diese Zahl durch die Zahl der überlebenden aus der mit der Referenzsubstanz behandelten Gruppe dividiert. Der so erhaltene Zahlenwert (Trimethoprimfaktor) ist ein Maß für die Wirkung der erfindungsgemäßen Substanzen im Vergleich zum Trimethoprim. F = 2 bedeutet also, daß die Substanz doppelt so wirksam ist wie Trimethoprim. Aus der folgenden Tabelle geht eine Überlegenheit der erfindungsgemäßen Substanzen gegenüber dem Trimethoprim bis zum 5-fachen hervor.

TABELLE

Allgemeine Formel I

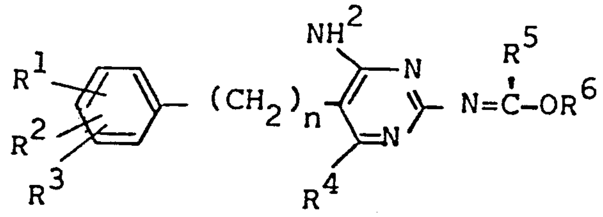

(I)

| Nr. | $R^1$ | $R^2$ | $R^3$ | $R^4$ | $R^5$ | $R^6$ | n | F |
|---|---|---|---|---|---|---|---|---|
| 1 | 4-Cl | H | H | $C_2H_5$— | $CH_3$— | $CH_3$— | 0 | 2,1 |
| 2 | 4-Cl | H | H | $C_2H_5$— | $CH_3$— | $C_2H_5$— | 0 | 5,0 |
| 3 | 4-Cl | H | H | $C_2H_5$— | $C_2H_5$— | $C_2H_5$— | 0 | 2,5 |
| 4 | 4-Cl | H | H | $C_2H_5$— | $C_3H_7$— | $C_2H_5$— | 0 | 2,4 |
| 5 | 4-Cl | H | H | H | $CH_3$— | $C_2H_5$— | 1 | 2,0 |
| 6 | 2-Cl | 4-Cl | H | H | $CH_3$— | $C_2H_5$— | 1 | 2,0 |
| 7 | 3-Cl | 4-Cl | H | H | $CH_3$— | $C_2H_5$— | 1 | 2,9 |
| 8 | 4-$CH_3$ | H | H | H | $CH_3$ | $C_2H_5$— | 1 | 1,7 |
| 9 | 4-$OCH_3$ | H | H | H | $CH_3$— | $C_2H_5$— | 1 | 1,25 |
| 10 | 3-$OCH_3$ | 4-$OCH_3$ | H | H | $CH_3$— | $C_2H_5$— | 1 | 2,0 |
| 11 | 3-$OCH_3$ | 4-$OCH_3$ | 5-$OCH_3$ | H | $CH_3$— | $CH_3$— | 1 | 2,6 |
| 12 | 3-$OCH_3$ | 4-$OCH_3$ | 5-$OCH_3$ | H | $CH_3$— | $C_2H_5$— | 1 | 1,3 |
| 13 | 3-$OCH_3$ | 4-$OCH_3$ | 5-$OCH_3$ | H | $C_2H_5$— | $C_2H_5$— | 1 | 1,4 |
| 14 | 3-$OCH_3$ | 4-$OCH_3$ | 5-$OCH_3$ | H | $C_3H_7$— | $C_2H_5$— | 1 | 1,5 |
| 15 | 3-$OCH_3$ | 4-$OCH_3$ | 5-$OCH_3$ | H | —$CH_2$—$C_6H_5$ | $C_2H_5$— | 1 | 1,5 |

Gegenstand der vorliegenden Erfindung sind demnach auch chemotherapeutische Mittel, die neben üblichen Träger- und Verdünnungsmitteln eine Verbindung der allgemeinen Formel I, insbesondere in Kombination mit einem Sulfonamid als Wirkstoffe enthalten, sowie die Verwendung der Verbindungen der allgemeinen Formel I als Sulfonamidpotentiatoren.

Die chemotherapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen.

## Beispiele 1

34 g 2,4-Diamino-5-(3,4,5-trimethoxybenzyl)-pyrimidin (Trimethoprim) werden mit 97,2 g Orthoessigsäuretriäthylester in 240 ml Dimethylformamid und 1 ml konzentrierter Salzsäure suspendiert. Nach 4-stündigem Rühren bei 80 bis 90°C behandelt man mit Aktivkohle, filtriert ab, engt im Vakuum ein und löst den Rückstand in der Wärme in 200 ml Butylacetat. Beim Abkühlen kristallisierten 35,2 g (81,5% d.Th.) N-[4-Amino-5-(3,4,5-trimethoxybenzyl)-pyrimidin-2-yl]-acetimidsäureäthylester mit dem Fp.: 142-144°C aus.

Analog Beispiel 1 wurden hergestellt:

2. N-[4-Amino-5-(3,4,5-trimethoxybenzyl)-pyrimidin-2-yl]-acetimidsäuremethylester mit dem Fp.: 171°C aus 2,4-Diamino-5-(3,4,5-trimethoxybenzyl)-pyrimidin und Orthoessigsäuretrimethylester.

3. N-[4-Amino-5-(3,4,5-trimethoxybenzyl)-pyrimidin-2-yl]-propionimidsäureäthylester mit dem Fp.: 137°C aus 2,4-Diamino-5-(3,4,5-trimethoxybenzyl)-pyrimidin und Orthopropionsäuretriäthylester.
4. N-[4-Amino-5-(3,4,5-trimethoxybenzyl)-pyrimidin-2-yl]-butyrimidsäureäthylester mit dem Fp.: 128°C aus 2,4-Diamino-5-(3,4,5-trimethoxybenzyl)-pyrimidin und Orthobuttersäuretriäthylester.
5. N-[4-Amino-5-(3,4,5-trimethoxybenzyl)-pyrimidin-2-yl]-isobutyrimidsäureäthylester mit dem Fp.: 136°C aus 2,4-Diamino-5-(3,4,5-trimethoxybenzyl)-pyrimidin und Orthoisobuttersäuretriäthylester.
6. N-[4-Amino-5-(3,4,5-trimethoxybenzyl)-pyrimidin-2-yl]-phenylacetimidsäureäthylester mit dem Fp.: 168°C aus 2,4-Diamono-5-(3,4,5-trimethoxybenzyl)-pyrimidin und Orthophenylessigsäuretriäthylester.
N-[4-Amino-5-(4-methoxybenzyl)-pyrimidin-2-yl]-acetimidsäureäthylester mit dem Fp.: 126°C aus 2,4-Diamino-5-(4-methoxybenzyl)-pyrimidin und Orthoessigsäuretriäthylester.
8. N-[4-Amino-5-(3,4-dimethoxybenzyl)-pyrimidin-2-yl]-acetimidsäureäthylester mit dem Fp.: 148°C aus 2,4-Diamino-5-(3,4-dimethoxybenzyl)-pyrimidin und Orthoessigsäuretriäthylester.
9. N-[4-Amino-5-(4-methylbenzyl)-pyrimidin-2-yl]-acetimidsäureäthylester mit dem Fp.: 129°C aus 2,4-Diamino-5-(4-methylbenzyl)-pyrimidin und Orthoessigsäuretriäthylester.
10. N-[4-Amino-5-(4-chlorbenzyl)-pyrimidin-2-yl]-acetimidsäureäthylester mit dem Fp.: 154°C aus 2,4-Diamino-5-(4-chlorobenzyl)-pyrimidin und Orthoessigsäuretriäthylester.
11. N-[4-Amino-5-(3,4-dichlorbenzyl)-pyrimidin-2-yl]-acetimidsäureäthylester mit dem Fp.: 158°C aus 2,4-Diamino-5-(3,4-dichlorbenzyl)-pyrimidin und Orthoessigsäuretriäthylester.
12. N-[4-Amino-5-(2,4-dichlorbenzyl)-pyrimidin-2-yl]-acetimidsäureäthylester mit dem Fp.: 128°C aus 2,4-Diamino-5-(2,4-dichlorbenzyl)-pyrimidin und Orthoessigsäuretriäthylester.

Beispiel 13

29,85 g 5-(4-Chlorphenyl)-6-äthyl-2,4-diamino-pyrimidin (Pyrimethamin) werden mit 97,32 g Orthoessigsäuretriäthylester in 240 ml Dimethylformamid und 1 ml konzentrierter Salzsäure suspendiert. Nach 4-stündigem Rühren bei 85 bis 100°C wird die klare Reaktionslösung mit Aktivkohle behandelt, filtriert und im Vakuum eingeengt. Nach Lösen in 100 ml Isopropyläther in der Wärme kristallisieren 32,8 g (85,2% der Theorie) N-[4-Amino-6-äthyl-5-(4-chlorphenyl)-pyrimidin-2-yl]-acetimidsäureäthylester mit dem Fp.: 143 bis 145°C aus.

Analog Beispiel 13 wurden hergestellt:

14. N-[4-Amino-6-äthyl-5-(4-chlorphenyl)-pyrimidin-2-yl]-acetimidsäuremethylester mit dem Fp.: 146°C aus 5-(4-Chlorphenyl)-6-äthyl-2,4-diamino-pyrimidin und Orthoessigsäuretrimethylester.

15. N-[4-Amino-6-äthyl-5-(4-chlorphenyl)-pyrimidin-2-yl]-propionimidsäureäthylester mit dem Fp.: 124°C aus 5-(4-Chlorphenyl)-6-äthyl-2,4-diamino-pyrimidin und Orthopropionsäuretriäthylester.

16. N-[4-Amino-6-äthyl-5-(4-dichlorphenyl)-pyrimidin-2-yl]-butyrimidsäureäthylester mit dem Fp. 129°C aus 5-(4-Chlorphenyl)-6-äthyl-2,4- diaminopyrimidin und Orthobuttersäuretriäthylester.

17. N-[4-Amino-6-äthyl-5-(4-chlorphenyl)-pyrimidin-2-yl]-phenylacetimidsäureäthylester mit dem Fp.: 120°C aus 5-(4-Chlorphenyl)-6-äthyl-2,4-diaminopyrimidin und Orthophenylessigsäuretriäthylester.

Beispiel für Zubereitungen

18. 400 mg 2-Sulfanilamido-4,5-dimethyloxazol

80 mg N-[4-Amino-5-(3,4,5-trimethoxybenzyl)-pyrimidin-2-yl]-acetamidsäuremethylester (Biespiel 2)

20 mg Maisstärke

10 mg Gelatine

8 mg Talkum

2 mg Magnesiumstearat

20 mg Primojel

Die Wirstoffe werden mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

19. 160 mg 2-Sulfanilamido-5-methoxy-pyrimidin
80 mg N-[(4-Amino-5-(3,4,5-trimethoxybenzyl)-pyrimidin-2-yl]-acetamidsäureäthylester (Beispiel 1)

5 mg Gelatine

30 mg Maisstärke

4 mg Talkum

1 mg Magnesiumstearat

Die Wirkstoffe werden mit wäßriger Gelatinelösung granuliert und nach dem Trocknen mit Maisstärke, Talkum und Magnesiumstearat vermischt. Aus dieser Mischung werden in üblicher Weise Tabletten gepreßt.

20. 400 mg 2-Sulfanilamido-4,5-dimethyloxazol

80 mg N-[4-amino-6-äthyl-5-(4-chlorphenyl)-pyrimidin-2-yl]-acetamidsäureäthylester (Beispiel 13)

20 mg Maisstärke

10 mg Gelatine

8 mg Talkum

2 mg Magnesiumstearat

20 mg Primojel

Die Wirkstoffe werden mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

21. 4,00 g 2-Sulfanilamido-5-methoxy-pyrimidin
2,00 g N-[4-Amino-5-(3,4,5-trimethoxybenzyl)-pyrimidin-2-yl]-acetamidsäuremethylester (Beispiel 2)

1,9 g Tylose C 30

30,0 g Zucker

10,0 g Glycerin

2,5 g Bentonit

0,06 g Aroma

0,04 g Nipagin M

0,06 g Nipasol-Natrium

ad 100,00 g demineralisiertes Wasser

Die feinst gemahlenen Wirkstoffe werden in dem wäßrigen Tylose-Schleim suspendiert. Anschließend werden alle anderen Bestandteile unter Rühren nacheinander zugegeben. Zum Schluß wird mit Wasser auf 100,0 g aufgefüllt.

**Patentansprüche**

1. N-Pyrimidinyl-imidsäureester der allgemeinen Formel I

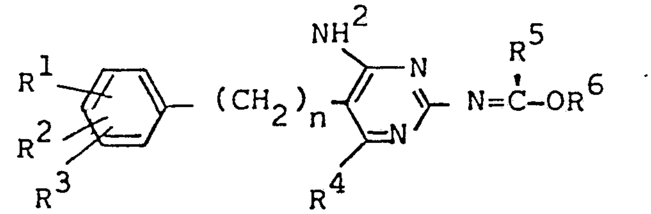

(I)

in der $R^1$, $R^2$ und $R^3$, die gleich oder verschieden voneinander sein können, Wasserstoff, Methyl, Methoxy oder Chlor bedeuten, $R^4$ Wasserstoff oder Alkyl mit 1—4 C-Atomen bedeutet, $R^5$ Alkyl mit 1—6 C-Atomen oder Benzyl bedeutet, $R^6$ Alkyl mit 1—4 C-Atomen oder Benzyl bedeutet, und n gleich 0 oder 1 sein kann.

2. N-[4-Amino-(3,4,5-trimethoxybenzyl)-pyrimidin-2-yl]-acetimidsäuremethylester.

3. N-[4-Amino-5-(3,4-dimethoxybenzyl)-pyrimidin-2-yl]-acetimidsäureäthylester.

4. N-[4-Amino-6-äthyl-5-(4-chlorphenyl)-pyrimidin-2-yl]-acetimidsäureäthylester.

5. N-[4-Amino-6-äthyl-5-(4-chlorphenyl)-pyrimidin-2-yl]-acetimidsäuremethylester.

6. N-[4-Amino-6-äthyl-5-(4-chlorphenyl)-pyrimidin-2-yl]-propionimidsäureäthylester.

7. Verfahren zur Herstellung der Verbindungen gemäß Ansprüchen 1 bis 6, dadurch gekennzeichnet, daß man eine Verbindung der allgemeinen Formel II

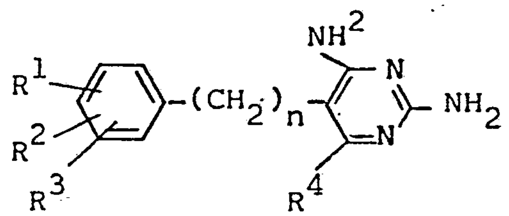

(II)

worin $R^1$, $R^2$, $R^3$, $R^4$ und n die gleiche Bedeutung wie im Anspruch 1 haben, mit einem Orthocarbonsäureester der allgemeinen Formel III

$$R^5—C(OR^6)_3 \qquad \text{(III)}$$

worin $R^5$ und $R^6$ die gleiche Bedeutung wie im Anspruch 1 haben, gegebenenfalls in Gegenwart eines Lösungsmittels und katalytischer Mengen einer Säure umsetzt.

8. Arzneimittel, enthaltend eine Verbindung gemäß Ansprüchen 1 bis 6, gegebenenfalls zusammen mit einem Sulfonamid, und nicht-toxischen, therapeutisch verträglichen festen oder flüssigen Trägerstoffen und galenischen Hilfsmitteln.

**Claims**

1. N-pyrimidinyl-imidoacid esters of the general formula I

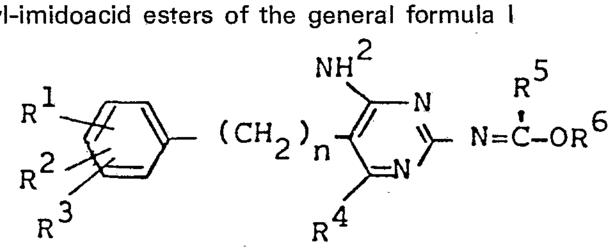

(I)

where $R^1$, $R^2$ and $R^3$, which may be identical or different, are hydrogen, methyl, methoxy or chlorine, $R^4$ is hydrogen or alkyl or 1 to 4 carbon atoms, $R^5$ is alkyl of 1 to 6 carbon atoms or benzyl, $R^6$ is alkyl of 1 to 4 carbon atoms or benzyl and n is 0 or 1.

2. N-[4-Amino-5-(3,4,5-trimethoxybenzyl)-pyrimidin-2-yl]-acetimido-acid methyl ester.

3. N-[4-Amino-5-(3,4-dimethoxybenzyl)-pyrimidin-2-yl]-acetimido-acid ethyl-ester.

4. N-[4-Amino-6-ethyl-5-(4-chlorphenyl)-pyrimidin-2-yl]-acetimido-acid ethyl ester.

5. N-[4-Amino-6-ethyl-5-(4-chlorophenyl)-pyrimidin-2-yl]-acetimido-acid methyl ester.

6. N-[4-Amino-6-ethyl-5-(4-chlorophenyl)-pyrimidin-2-yl]- propionimido-acid ethyl ester.

7. A process for the preparation of a compound as claimed in claims 1 to 6, characterized in that a compound of the general formula II

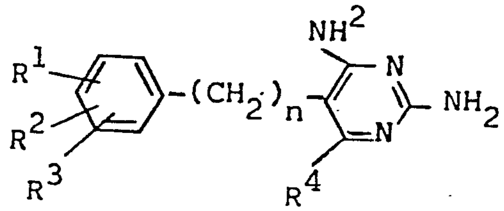

(II)

where $R^1$, $R^2$, $R^3$, $R^4$ and n have the same meanings as in formula I in claim 1, is reacted with an orthocarboxylic acid ester of the formula III

$$R^5—C(OR^6)_3 \quad \text{(III)}$$

where $R^5$ and $R^6$ have the same meanings as in formula I in claim 1, in the presence or absence of a solvent and of a catalytic amount of an acid.

8. A drug which contains a compound as claimed in claims 1 to 6, with or without a sulfonamide and non-toxic, therapeutically acceptable solid or liquid carriers and galenical assistants.

**Revendications**

1. Ester de N-pyrimidinyl-imido-acide de formule générale

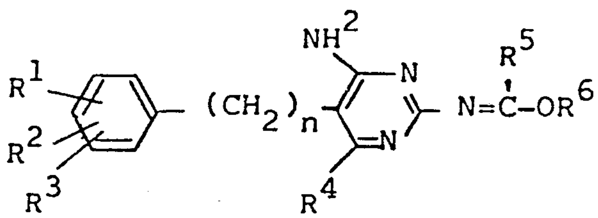

(I)

dans laquelle $R^1$, $R^2$ et $R^3$, qui peuvent être identiques ou différents les uns des autres, représentent hydrogène, méthyle, méthoxy, ou chlore, $R^4$ hydrogène ou alkyle en $C_1$ à $C_4$, $R^5$ alkyle en $C_1$ à $C_6$ ou benzyle, $R^6$ alkyle en $C_1$ à $C_4$ ou benzyle et *n* est 0 ou 1.

2. Ester méthylique de N-[4-amino-5-(3,4,5-triméthoxybenzyl)-pyrimidin-2-yl]-acétimido-acide.

3. Ester éthylique de N-[4-amino-5-(3,4-diméthoxybenzyl)-pyrimidin-2-yl]-acétimido-acide.

4. Ester éthylique de N-[4-amino-6-éthyl-5-(4-chlorophényl)-pyrimidin-2-yl]-acétimido-acide.

5. Ester méthylique de N-[4-amino-6-éthyl-5-(4-chlorophényl)-pyrimidin-2-yl]-acétimido-acide.

6. Ester éthylique de N-[4-amino-6-éthyl-5-(4-chlorophényl)-pyrimidin-2-yl]-propionimido-acide.

7. Procédé de préparation de composés selon l'une des revendications 1 à 6, caractérisé par le fait qu'on fait réagir un composé de formule générale II

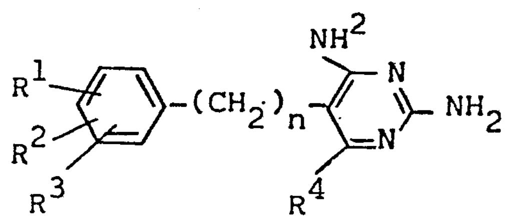

(II)

dans laquelle $R^1$, $R^2$, $R^3$, $R^4$ et *n* ont les mêmes significations que dans la formule I de la revendication 1, avec un ester d'acide orthocarboxylique de formule III

$$R^5—C(OR^6)_3 \quad \text{(III)}$$

dans laquelle $R^5$ et $R^6$ ont la même signification que dans la formule I de la revendication 1, éventuellement en présence d'un solvant et de quantités catalytiques d'un acide.

8. Médicament contenant un composé selon l'une des revendications 1 à 6, éventuellement, en même temps qu'un sulfonamide, des supports solides ou liquides, non toxiques et compatibles thérapeutiquement et des adjuvants galéniques.